(19) Europäisches Patentamt
European Patent Office
Office européen des brevets

(11) EP 1 989 172 B1

(12) # EUROPÄISCHE PATENTSCHRIFT

(45) Veröffentlichungstag und Bekanntmachung des Hinweises auf die Patenterteilung:
**21.09.2011 Patentblatt 2011/38**

(21) Anmeldenummer: **07704599.5**

(22) Anmeldetag: **15.02.2007**

(51) Int Cl.:
***C07C 209/02*** *(2006.01)*

(86) Internationale Anmeldenummer:
**PCT/EP2007/051473**

(87) Internationale Veröffentlichungsnummer:
**WO 2007/096297 (30.08.2007 Gazette 2007/35)**

(54) **DIREKTAMINIERUNG VON KOHLENWASSERSTOFFEN**

DIRECT AMINATION OF HYDROCARBONS

AMINATION DIRECTE D'HYDROCARBURES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **24.02.2006 EP 06110416**

(43) Veröffentlichungstag der Anmeldung:
**12.11.2008 Patentblatt 2008/46**

(73) Patentinhaber: **BASF SE**
**67056 Ludwigshafen (DE)**

(72) Erfinder:
- **VAN LAAR, Frederik**
  **Dubai (AE)**
- **SCHWAB, Ekkehard**
  **67434 Neustadt (DE)**
- **ANDERS, Joachim-Thierry**
  **67161 Gönnheim (DE)**
- **CRONE, Sven**
  **67117 Limburgerhof (DE)**
- **HÖLEMANN, Karl**
  **68199 Mannheim (DE)**
- **MACKENROTH, Wolfgang**
  **67098 Bad Dürkheim (DE)**
- **KUBANEK, Petr**
  **68163 Mannheim (DE)**

(56) Entgegenhaltungen:
**WO-A-01/32600** **WO-A-99/10311**
**WO-A-2007/025882** **CA-A- 553 998**

- **HAGEMEYER A ET AL: "Application of combinatorial catalysis for the direct amination of benzene to aniline" APPLIED CATALYSIS A: GENERAL, ELSEVIER SCIENCE, AMSTERDAM, NL, Bd. 227, Nr. 1-2, 8. März 2002 (2002-03-08), Seiten 43-61, XP004340850 ISSN: 0926-860X**

Bemerkungen:
Die Akte enthält technische Angaben, die nach dem Eingang der Anmeldung eingereicht wurden und die nicht in dieser Patentschrift enthalten sind.

EP 1 989 172 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft ein Verfahren zur bevorzugt kontinuierlichen Aminierung, bevorzugt Direktaminierung von Kohlenwasserstoffen bevorzugt durch Umsetzung von Kohlenwasserstoffen, besonders bevorzugt aromatischen Kohlenwasserstoffen, insbesondere Benzol, in einem ersten Reaktionsraum mit Ammoniak in Gegenwart von Katalysator (i), der die Aminierung katalysiert, und danach das Oxidationsmittel dem Reaktionsgemisch zuführt, wobei man Oxidationsmittel, bevorzugt Luft, Sauerstoff, CO, $CO_2$, NO und/oder $N_2O$, besonders bevorzugt Sauerstoff, dem Reaktionsgemisch zuführt und in einem anschließenden Reaktionsraum das Oxidationsmittel mit Wasserstoff, das bei der Aminierung entsteht, in Gegenwart eines Katalysators (ii) umsetzt, der diese Umsetzung mit Wasserstoff katalysiert.

Die Kohlenwasserstoffe können beliebige Kohlenwasserstoffe sein, wie aromatische Kohelnwasserstoffe, aliphatische Kohlenwasserstoffe und cycloaliphatische Kohlenwasserstoffe, die beliebig substituiert sein können und innerhalb ihrer Kette bzw. ihres Rings oder ihrer Ringe Heteroatome und Doppel- oder Dreifachbindungen aufweisen können,

Insbesondere bezieht sich die Erfindung auf Verfahren zur Aminierung von Kohlenwasserstoffen bevorzugt durch Umsetzung von aromatischen Kohlenwasserstoffen, besonders bevorzugt Benzol mit Ammoniak insbesondere gemäß der folgenden Reaktion, die bevorzugt katalysiert ist:

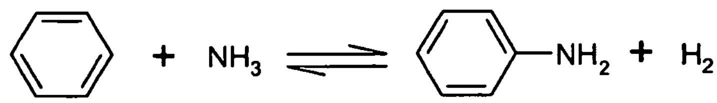

Die kommerzielle Herstellung von Aminen, insbesondere von aromatischen Aminen, wie Anilin, wird üblicherweise in mehrstufigen Reaktionen durchgeführt. Anilin wird beispielsweise üblicherweise durch Umwandlung von Benzol in ein Benzol-Derivat, z.B. Nitrobenzol, Chlorbenzol oder Phenol und anschließende Umwandlung dieses Derivats in Anilin hergestellt.

Vorteilhafter als solche indirekten Verfahren zur Herstellung von insbesondere aromatischen Aminen sind Methoden, die eine direkte Herstellung der Amine aus den entsprechenden Kohlenwasserstoffen ermöglichen. Eine sehr elegante Route ist die heterogenkatalysierte Direktaminierung von Benzol, erstmals 1917 von Wibaut (Berichte, 50, 541-546) beschrieben. Da die Direktaminierung gleichgewichtslimitiert ist, wurden mehrere Systeme beschrieben, welche die Gleichgewichtslimitierung durch die selektive Entfernung von Wasserstoff aus der Reaktion verschiebt und einen erhöhten Benzolumsatz ermöglicht. Die meisten Verfahren basieren auf dem Einsatz von Metalloxiden, welche durch Wasserstoff reduziert werden, damit den Wasserstoff aus dem Reaktionssystem entfernen und somit das Gleichgewicht verschieben.

CN 1555921A offenbart die Oxido-aminierung von Benzol in der Flüssigphase, wobei als "O"-Donor Wasserstoffperoxyd fungiert. Die Anwendung von $H_2O_2$ ist allerdings aufgrund des Preises und der geringen Selektivität aufgrund von Folgereaktionen für Massenchemikalien nur bedingt geeignet.

In CA 553,988 ist ein Verfahren zur Herstellung von Anilin aus Benzol offenbart, worin Benzol, Ammoniak und gasförmiger Sauerstoff bei einer Temperatur von etwa 1000°C an einem Platinkatalysator umgesetzt werden. Geeignete Platin enthaltende Katalysatoren sind Platin allein, Platin mit bestimmten spezifischen Metallen und Platin zusammen mit bestimmten spezifischen Metalloxiden. Des weiteren ist in CA 553,988 ein Verfahren zur Herstellung von Anilin offenbart, worin Benzol in der Gasphase mit Ammoniak in Anwesenheit eines reduzierbaren Metalloxids bei Temperaturen von 100 bis 1000°C umgesetzt wird, ohne Zugabe von gasförmigem Sauerstoff. Geeignete reduzierbare Metalloxide sind die Oxide des Eisens, Nickels, Kobalts, Zinns, Antimons, Bismuts und Kupfers.

US 3,919,155 betrifft die Direktaminierung von aromatischen Kohlenwasserstoffen mit Ammoniak, wobei als Katalysator Nickel/Nickeloxid eingesetzt wird, wobei der Katalysator zusätzlich Oxide und Carbonate von Zirkonium, Strontium, Barium, Calcium, Magnesium, Zink, Eisen, Titan, Aluminium, Silizium, Cer, Thorium, Uran und Alkalimetallen enthalten kann.

US 3,929,889 bezieht sich ebenfalls auf die Direktaminierung von aromatischen Kohlenwasserstoffen mit Ammoniak an einem Nickel/Nickeloxidkatalysator, wobei der eingesetzte Katalysator teilweise zu elementarem Nickel reduziert wurde und anschließend reoxidiert wurde um einen Katalysator zu erhalten, der ein Verhältnis von Nickel : Nickeloxid von 0,001 : 1 bis 10 : 1 aufweist.

US 4,001,260 offenbart ein Verfahren zur Direktaminierung von aromatischen Kohlenwasserstoffen mit Ammoniak, wobei wiederum ein Nickel/Nickeloxidkatalysator eingesetzt wird, der auf Zirkoniumdioxid aufgebracht ist, und vor Einsatz in der Aminierungsreaktion mit Ammoniak reduziert wurde.

US 4,031,106 betrifft wiederum die Direktaminierung von aromatischen Kohlenwasserstoffen mit Ammoniak an einem Nickel/Nickeloxidkatalysator auf einem Zirkoniumdioxidträger, der weiterhin ein Oxid, ausgewählt aus Lanthanoiden und Seltenerdmetallen, enthält.

DE 196 34 110 beschreibt die nicht oxidative Aminierung bei einem Druck von 10 - 500 bar und einer Temperatur von 50-900°C, wobei die Umsetzung in Gegenwart eines sauren Heterogenkatalysators erfolgt, der mit leichten und schweren Platinmetallen modifiziert ist.

WO 00/09473 beschreibt ein Verfahren zur Herstellung von Aminen durch Direktaminierung von aromatischen Kohlenwasserstoffen an einem Katalysator, enthaltend mindestens ein Vanadiumoxid.

WO 99/10311 lehrt ein Verfahren zur Direktaminierung von aromatischen Kohlenwasserstoffen bei einer Temperatur von < 500°C und einem Druck von < 10 bar. Als Katalysator wird ein Katalysator enthaltend mindestens ein Metall ausgewählt aus Übergangsmetallen, Lanthaniden und Actiniden, bevorzugt Cu, Pt, V, Rh und Pd, eingesetzt. Bevorzugt wird die Direktaminierung zur Erhöhung der Selektivität und/oder des Umsatzes in Anwesenheit eines Oxidationsmittels durchgeführt.

WO 01/32600 offenbart einen Prozess für die direkte Aminierung von Benzol mit Amoniak in Gegewart von Sauerstoff und einem Katalysator bzw. Katalysatoren enthaltend ein reduzierbares Metalloxid wie z.B. CuO Osder NiO und ein Metall wie z.B. Platin, Palladium oder Silber, bei der gegebenenfalls zusätzlich Wasserstoff der Reaktion zugeführt werden muss.

WO 00/69804 betrifft ein Verfahren zur Direktaminierung von aromatischen Kohlenwasserstoffen, wobei als Katalysator ein Komplex eingesetzt wird, enthaltend ein Edelmetall und ein reduzierbares Metalloxid. Dabei sind Katalysatoren, enthaltend Palladium und Nickeloxid bzw. Palladium und Kobaltoxid, besonders bevorzugt.

Die meisten der genannten Verfahren gehen dabei von einem Mechanismus zur Direktaminierung aus, wie er in der Zusammenfassung von WO 00/69804 aufgeführt ist. Danach erfolgt zunächst die (edel)metallkatalysierte Herstellung der gewünschten Aminverbindung aus dem aromatischen Kohlenwasserstoff und Ammoniak und in einem zweiten Schritt das "Abfangen" des im ersten Schritt entstandenen Wasserstoffs mit einem reduzierbaren Metalloxid. Die gleichen mechanistischen Überlegungen werden dem Verfahren in WO 00/09473 zugrundegelegt, worin der Wasserstoff mit Sauerstoff aus Vanadiumoxiden abgefangen wird (Seite 1, Zeilen 30 bis 33). Der gleiche Mechanismus wird auch in US 4,001,260 zugrundegelegt, wie aus den Ausführungen und der Abbildung in Spalte 2, Zeilen 16 bis 44 ersichtlich ist.

Aufgabe der vorliegenden Erfindung ist es, ein besonders wirtschaftliches Verfahren zur Aminierung von Kohlenwasserstoffen, insbesondere Verfahren zur Umsetzung von Benzol mit Ammoniak zu entwickeln, bei dem ein bevorzugt kontinuierliches Verfahren bei möglichst hoher Selektivität und/oder möglichst hohem Umsatz ermöglicht wird. Diese Aufgabe wird dadurch gelöst, dass man Oxidationsmittel, bevorzugt Luft, Sauerstoff. CO, $CO_2$, NO und/oder $N_2O$, besonders bevorzugt Sauerstoff, dem Reaktionsgemisch zuführt und das Oxidationsmittel mit Wasserstoff, das bei der Aminierung entsteht, in Gegenwart eines Katalysators (ii) umsetzt, der diese Umsetzung mit Wasserstoff katalysiert.

Es wurde überraschenderweise gefunden, dass der Umsatz bei der Direktaminierung an Metallkatalysatoren (zum Beispiel Ni, Fe, Co, Cu, EM oder deren Legierungen, wobei EM für Edelmetalle steht), verglichen mit dem Gleichgewichtsumsatz, erheblich gesteigert werden konnte, wenn man den bei der Umsetzung des Kohlenwasserstoffs mit dem Ammoniak entstehenden Wasserstoff durch Einsatz von Oxidationsmittel und deren Umsetzung mit dem Wasserstoff in Gegenwart von entsprechenden Katalysatoren entfernt. Überraschend konnte festgestellt werden, dass man durch den Einsatz des Katalysators (ii) die Direktaminierung nicht negativ beeinflusst, durch die schnellere Entfernung des Wasserstoffs den Umsatz aber erhöht.

Bei den eingangs beschriebenen, bekannten metall-metalloxidischen Systemen muss der Katalo-reaktant nach einiger Zeit wieder mit "Sauerstoff" aufgeladen worden. Dies bedeutet kostenintensive Unterbrechungen, da die Aminierung und die Reaktivierung üblicherweise nicht bei den gleichen Bedingungen (Druck und Temperatur) ablaufen. Der Reaktor muss somit üblicherweise entspannt, gespült und inertisiert, der Katalysator reaktiviert, und wieder unter Reaktionsbedingungen gebracht werden. Die Selektivität der gesamten Reaktion ändert sich mit dem Säuerstoffinhalt des Kataloreaktants und muss also durch Verfahrensänderung (Druck, Ammoniak-Aromaten-Verhältnis und/oder Temperatur) kompensiert werden. Eine hinreichende Selektivität für die C als auch N Bilanz kann mit diesen Systemen nicht erreicht werden, da Ammoniak durch die Metalloxide oder durch adsorbierten Säuerstoff an der Oberfläche unter Bildung von $N_2$ und $H_2O$ verbrennt wird. Die Bereitstellung einer vollintegrierten Lösung ist somit mit metalloxidischen Systemen schwierig oder gar nicht zu bewerkstelligen.

Diese Nachteile werden durch die erfindungsgemäße Entfernung des Wasserstoffs aus dem Reaktionssystem vermieden. Das erfindungsgemäße Verfahren ermöglicht somit eine sehr effiziente, selektive, kostengünstige Direktaminierung.

Erfindungsgemäß wird Oxidationsmittel, bevorzugt Luft, Sauerstoff, CO, $CO_2$, NO und/oder $N_2O$ besonders bevorzugt Sauerstoff, dem Reaktionsgemisch zugeführt und mit Wasserstoff, das bei der Aminierung entsteht, in Gegenwart des Katalysators (ii) umgesetzt. Das Oxidationsmittel kann in dem erfindungsgemäßen Verfahren gegebenenfalls mit weiteren Coreaktanden, Cokatalysatoren oder weitere Reagenzien in die Reaktionszone des Reaktors gegeben werden, jeweils in Abhängigkeit von der durchgeführten Aminierung. Zum Beispiel kann bei der Aminierung von Benzol Sauerstoff oder ein Sauerstoff enthaltendes Gas in die Reaktionszone des Reaktors gegeben werden, als Coreaktand.

Die relative Menge des gasförmigen Sauerstoffs, der in die Reaktionszone gegeben werden kann, ist variabel und unter anderem von dem eingesetzten Katalysatorsystem abhängig. Das molare Verhältnis von gasförmigem Sauerstoff zu Benzol kann zum Beispiel im Bereich von 0,05 zu 1 bis 1 zu 1, bevorzugt 0,1 zu 1 bis 0,5 zu 1 liegen.

Erfindungsgemäß wird der bei der Aminierung entstehende Wasserstoff mit dem Oxidationsmittel umgesetzt, wobei lin der Verfahrensführung wird zunächst der Kohlenwasserstoff mit Ammoniak in Gegenwart des Katalysators (i) umgesetzt und anschließen das Oxidationsmittel dem Reaktionsgemisch zugeführt. Dabei kann das Oxidationsmittel zusammen mit dem Feed, d.h. dem Benzol und dem Ammoniak zugegeben werden. Bevorzugt wird das Oxidationsmittel jedoch erst nach einer ersten Aminierungszone an einer oder mehreren Stellen zugegeben.

Das erfindungsgemäße Verfahren erfolgt derart, dass man in einem ersten Reaktionsraum, z.B. einem Reaktor oder Teil eines Reaktors, Kohlenwasserstoff mit Ammoniak in Gegenwart des Katalysators (i) umsetzt, danach das Oxidationsmittel dem Reaktionsgemisch zuführt und in einem anschließende Reaktionsraum, z.B. einem Reaktor oder Teil eines Reaktors, das Oxidationsmittel mit dem bei der Aminierung entstehendem Wasserstoff in Gegenwart der Verbindung (ii), die diese Umsetzung mit Wasserstoff katalysiert, umsetzt, wobei man während oder nach der Wasserstoffoxidation Kohlenwasserstoff mit Ammoniak in Gegenwart des Katalysators (i) umsetzt. Dabei kann das Verfahren derart ausgestaltet sein, dass die Katalysatoren (i) und (ii) jeweils in getrennten Katalysatorbetten vorliegen, das Oxidationsmittel dem Reaktionsgemisch vor einem Katalysatorbett mit dem Katalysator (ii) zugeführt wird und auf das Katalysatorbett mit dem Katalysator (ii) ein Katalysatorbett mit dem Katalysator (i) folgt. Darauf kann wiederum ein Katalysatorbett mit dem Katalysator (ii) folgen.

Das erfindungsgemäße Verfahren, d.h. die Aminierung von Kohlenwasserstoffen, insbesondere die Umsetzung von Benzol mit Ammoniak wird in Gegenwart von Verbindungen durchgeführt, die die Aminierung katalysieren (Katalysatoren (i)).

Als Katalysatoren (i) können die für die direkte Aminierung von Kohlenwasserstoffen, insbesondere die für die direkte Aminierung von Benzol mit Ammoniak zu Anilin bekannten Katalysatoren eingesetzt werden. Derartige Katalysatoren sind in der Patentliteratur vielfältig beschrieben und allgemein bekannt. Da gemäß dem erfindungsgemäßen Verfahren eine Entfernung des Wasserstoffs durch Reaktion mit extra zugeführtem Oxidationsmittel erfolgt können auch Katalysatoren Verwendung finden, die keine gegenüber Wasserstoff reaktive Komponenten aufweisen. Als Katalysatoren kommen beispielsweise übliche Metallkatalysatoren in Frage, z.B. solche auf der Basis von Nickel, Eisen, Kobalt, Kupfer, Edelmetallen oder Legierungen dieser genannten Metalle. Als Edelmetalle (EM) können alle Edelmetalle in Betracht kommen, z.B. Ru, Rh, Pd, Ag, Ir, Pt und Au, wobei die Edelmetalle Ru und Rh bevorzugt nicht alleine eingesetzt werden, sondern in Legierung mit anderen Übergangsmetallen, beispielsweise Co, Cu, Fe und Nickel oder deren Gemische. Solche Legierungen werden auch bevorzugt eingesetzt bei Anwendung der anderen Edelmetallen, zum Beispiel sind geträgerte NiCuEM; CoCuEM; NiCoCuEM, NiMoEM, NiCrEM, NiReEM, CoMoEM, CoCrEM, CoReEM, FeCuEM, FeCoCuEM, FeMoEM, FeReEM Legierungen von Interesse, dabei ist EM ein Edelmetall, insbesondere bevorzugt Ag und/oder Au.

Der Katalysator kann in allgemein üblicher Form, z.B. als Pulver oder als in ein Festbett einsetzbares System eingesetzt werden (wie beispielsweise Stränge, Kugeln, Tabletten, Ringe, wobei die katalytisch aktiven Bestandteile gegebenenfalls auf einem Trägermaterial vorliegen können. Als Trägermaterial kommen z.B. anorganische Oxide, beispielsweise $ZrO_2$ $SiO_2$, $Al_2O_3$, MgO, $TiO_2$, $B_2O_3$, CaO, ZnO, BaO, $ThO_2$, $CeO_2$, $Y_2O_3$ und Mischungen dieser Oxide, z.B. Magnesium-Aluminium-oxid, bevorzugt $TiO_2$, $ZrO_2$, $Al_2O_3$, Magnesium-Aluminium-oxid und $SiO_2$, besonders bevorzugt $ZrO_2$ und Magnesium-Aluminium-oxid in Frage. Unter $ZrO_2$ wird sowohl reines $ZrO_2$ als auch das normale Hf-enthaltende $ZrO_2$ verstanden.

Die in dem erfindungsgemäßen Verfahren bevorzugt eingesetzten Katalysatoren können regeneriert werden, z.B. indem man eine reduktive (beispielsweise $H_2$-Atmosphäre) über den Katalysator leitet oder zuerst eine oxidative und anschließend eine reduktive Atmosphäre über oder durch das Katalysatorbett führt.

Bevorzugt wird man als Katalysator (i) Verbindungen einsetzen, die Ni, Co, Fe, Cu oder Kombinationen dieser Elemente enthalten, bevorzugt setzt man als Katalysator (i) Verbindungen ein, die Ni-Cu-X, Fe-Cu-X und/oder Co-Cu-X enthalten, wobei X Ag oder Mo darstellt, besonders bevorzugt die Kombination Ni-Cu und/oder Fe-Cu, insbesondere deren Kombinationen mit zusätzlichem Dotierelement Ni-Cu-X, Fe-Cu-X, wobei X Ag, oder Mo darstellt. Insbesondere bevorzugt sind Legierungen von NiCu(Ag oder Mo) und/oder FeCu(Ag oder Mo). Dabei können die Katalysatoren, d.h. die Elemente im Katalysator (i) in reduzierter sowie auch in oxidierter Form vorliegen.

Bevorzugt beträgt in dem Katalysator (i) der Gewichtsanteil der Elemente Ni, Co und Fe zusammen, d.h. der Anteil des Gesamtgewichts dieser Elemente, wobei nicht alle Elemente in dem Katalysator vorhanden sein müssen, zwischen 0,1 Gew.% und 75 Gew.-%, besonders bevorzugt zwischen 1 Gew.-% und 70 Gew.-%, insbesondere zwischen 2 Gew.-% und 50 Gew.% und der Gewichtsanteil an Cu zwischen 0,1 Gew.-% und 75 Gew.-%, bevorzugt zwischen 0,1 Gew.-% und 25 Gew.-%, besonders bevorzugt zwischen 0,1 Gew.-% und 20 Gew.-%, insbesondere zwischen 2,5 Gew.-% und 10 Gew.-%, bezogen auf das Gesamtgewicht des Katalysators (i). Daneben kann der Katalysator (i) Trägermaterial enthalten.

Der Gewichtsanteil des Dotierelements X an dem Gesamtgewicht des Katalysators (i) beträgt bevorzugt zwischen 0,01 Gew.-% und 8 Gew.-%, besonders bevorzugt zwischen 0,1 Gew.-% und 5 Gew.-%, insbesondere zwischen 0,5 Gew.-% und 4 Gew.-%.

Bevorzugt werden die Katalysatorbetten enthaltend Katalysatoren (i) oder (ii) mit 0,1 bis 5, bevorzugt von 0,2 bis 2, insbesondere von 0,3 bis 1,5 kg Kohlenwasserstoff pro Liter Katalysatorbett und pro Stunde belastet. Bevorzugt unterscheiden sich die Katalysatoren (i) und (ii), besonders bevorzugt unterscheiden sich die Katalysatoren (i) und (ii) stofflich, insbesondere enthält der Katalysator (i) Elemente, die der Katalysator (ii) nicht enthält.

Erfindungsgemäß werden ein oder mehrere Katalysatoren (ii) eingesetzt, um den bei der Aminierung entstehenden Wasserstoff aus dem Reaktionssystem zu entfernen. Dabei kann man allgemein übliche und bekannte Katalysatoren einsetzen, die die Oxidation von Wasserstoff katalysieren, bevorzugt solche, die die Umsetzung von Sauerstoff mit Wasserstoff katalysieren. Derartige Katalysatoren sind allgemein bekannt und vielfältig beschrieben. Bevorzugt setzt man als Katalysator (ii) Pt und/oder Pd/Ag enthaltende Verbindungen ein, besonders bevorzugt solche, die Pt und/oder Pd/Ag, d.h. Pd und Ag, auf einem Träger enthalten, insbesondere reinem $SiO_2$, reinem $Al_2O_3$, $ZrO_2$ und/oder reinem $TiO_2$ als Träger. Als "rein" werden solche Träger verstanden, die Verunreinigungen <0,5 Gewichts-% enthalten. Besonders bevorzugt beträgt in dem Katalysator (ii) der Gewichtsanteil an Pt zwischen 0,0001 Gew.-% und 1 Gew.-%, bevorzugt zwischen 0,001 Gew.-% und 0,5 Gew.-%, insbesondere zwischen 0,01 Gew.-% und 0,1 Gew.-%, bezogen auf das Gesamtgewicht des Katalysators.

Mit dem erfindungsgemäßen Aminierungsverfahren können beliebige Kohlenwasserstoffe, wie aromatische Kohlenwasserstoffe, aliphatische Kohlenwasserstoffe und cycloaliphatische Kohlenwasserstoffe aminiert werden, die beliebig substituiert sein können und innerhalb ihrer Kette bzw. ihres Rings/ihrer Ringe Heteroatome und Doppel- oder Dreifachbindungen aufweisen können. Bevorzugt werden in dem erfindungsgemäßen Aminierungsverfahren aromatische Kohlenwasserstoffe und heteroaromatische Kohlenwasserstoffe eingesetzt. Die entsprechenden Produkte sind die korrespondierenden Arylamine oder Heteroarylamine.

Unter einem aromatischen Kohlenwasserstoff ist im Sinne der vorliegenden Erfindung ein ungesättigter cyclischer Kohlenwasserstoff zu verstehen, der einen oder mehrere Ringe aufweist und ausschließlich aromatische C-H-Bindungen enthält. Bevorzugt weist der aromatische Kohlenwasserstoff einen oder mehrere 5- oder 6-gliedrige Ringe auf.

Unter einem heteroaromatischen Kohlenwasserstoff sind solche aromatischen Kohlenwasserstoffe zu verstehen, in denen einer oder mehrere der Kohlenstoffatome des aromatischen Rings durch ein Heteroatom ausgewählt aus N, O und S ersetzt ist/sind.

Die aromatischen Kohlenwasserstoffe bzw. die heteroaromatischen Kohlenwasserstoffe können substituiert oder unsubstituiert sein. Unter einem substituierten aromatischen bzw. heteroaromatischen Kohlenwasserstoff sind Verbindungen zu verstehen, in denen ein oder mehrere Wasserstoffatome, die an ein Kohlenstoff- oder Heteroatom des aromatischen Rings gebunden sind/ist, durch einen anderen Rest ausgetauscht ist/sind. Solche Reste sind beispielsweise substituierte oder unsübstituierte Alkyl-, Alkenyl-, Alkinyl-, Heteroalkyl-, Heteroalkenyl-, Heteroalkinyl-, Cycloalkyl- und/oder Cycloalkinylreste. Des weiteren kommen die folgenden Reste in Frage: Halogen, Hydroxy, Alkoxy, Aryloxy, Amino, Amido, Thio und Phosphino. Bevorzugte Reste der aromatischen bzw. heteroaromatischen Kohlenwasserstoffe sind ausgewählt aus $C_{1-6}$-Alkyl, $C_{1-6}$-Alkenyl, $C_{1-6}$-Alkinyl, $C_{3-8}$-Cycloalkyl, $C_{3-8}$-Cycloalkenyl, Alkoxy, Aryloxy, Amino und Amido, wobei sich die Angabe $C_{1-6}$ auf die Anzahl der Kohlenstoffatome in der Hauptkette des Alkylrests, des Alkenylrests oder des Alkinylrests bezieht und die Bezeichnung $C_{3-8}$ auf die Anzahl der Kohlenstoffatome des Cycloalkyl- bzw. Cycloalkenylrings. Es ist weiterhin möglich, dass die Substituenten (Reste) des substituierten aromatischen oder heteroaromatischen Kohlenwasserstoffs weitere Substituenten aufweisen.

Die Zahl der Substituenten (Reste) des aromatischen bzw. heteroraromatischen Kohlenwasserstoffs ist beliebig. In einer bevorzugten Ausführungsform weist der aromatische bzw. heteroaromatische Kohlenwasserstoff jedoch mindestens ein Wasserstoffatom auf, das direkt an ein Kohlenstoffatom oder ein Heteroatom des aromatischen Rings gebunden ist. Somit weist ein 6-gliedriger Ring bevorzugt 5 oder weniger Substituenten (Reste) auf und ein 5-gliedriger Ring bevorzugt 4 oder weniger Substituenten (Reste). Besonders bevorzugt trägt ein 6-gliedriger aromatischer bzw. heteroaromatischer Ring 4 oder weniger Substituenten, ganz besonders bevorzugt 3 oder weniger Substituenten (Reste). Ein 5-gliedriger aromatischer oder heteroaromatischer Ring trägt bevorzugt 3 oder weniger Reste, besonders bevorzugt 2 oder weniger Reste.

In einer besonders bevorzugten Ausführungsform des erfindungsgemäßen Verfahrens wird ein aromatischer bzw. heteroaromatischer Kohlenwasserstoff der allgemeinen Formel

$$(A)-(B)_n$$

eingesetzt, worin die Symbole die folgende Bedeutung haben:

A unabhängig Aryl oder Heteroaryl, bevorzugt ist A ausgewählt aus Phenyl, Diphenyl, Diphenylmethan, Benzyl,

Dibenzyl, Naphthyl, Anthracen, Pyridyl und Chinolin;

n eine Zahl von 0 bis 5, bevorzugt 0 bis 4, insbesondere in dem Fall, wenn A ein 6-gliedriger Aryl- oder Heteroaryl-Ring ist; für den Fall, dass A ein 5-gliedriger Aryl- oder Heteroaryl-Ring ist, ist n bevorzugt 0 bis 4; unabhängig von der Ringgröße ist n besonders bevorzugt 0 bis 3, ganz besonders bevorzugt 0 bis 2 und insbesondere 0 bis 1; dabei tragen die übrigen, keine Substituenten B tragenden Kohlenwasserstoff- oder Heteroatome von A Wasserstoffatome oder gegebenenfalls keinen Substituenten;

B ist unabhängig voneinander ausgewählt aus der Gruppe bestehend aus Alkyl, Alkenyl, Alkinyl, substituiertem Alkyl, substituiertem Alkenyl, substituiertem Alkinyl, Heteroalkyl, substiuiertem Heteroalkyl, Heteroalkenyl, substituiertem Heteroalkenyl, Heteroalkinyl, substituiertem Heteroalkinyl, Cycloalkyl, Cycloalkenyl, substituiertem Cycloalkyl, substituiertem Cycloalkenyl, Halogen, Hydroxy, Alkoxy, Aryloxy, Carbonyl, Amino, Amido, Thio und Phosphino; bevorzugt ist B unabhängig voneinander ausgewählt aus $C_{1-6}$-Alkyl, $C_{1-6}$-Alkenyl, $C_{1-6}$-Alkinyl, $C_{3-8}$-Cycloalkyl, $C_{3-8}$-Cycloalkenyl, Alkoxy, Aryloxy, Amino und Amido.

Der Ausdruck unabhängig voneinander bedeutet, dass, wenn n 2 oder größer ist, die Substituenten B gleiche oder verschiedene Reste aus den genannten Gruppen sein können.

Unter Alkyl sind gemäß der vorliegenden Anmeldung verzweigte oder unverzweigte, gesättigte acyclische Kohlenwasserstoff-Reste zu verstehen. Beispiele für geeignete Alkyl-Reste sind Methyl, Ethyl, n-Propyl, i-Propyl, n-Butyl, t-Butyl, i-Butyl usw.. Bevorzugt werden Alkyl-Reste mit 1 bis 50 Kohlenstoffatomen, besonders bevorzugt mit 1 bis 20 Kohlenstoffatomen, ganz besonders bevorzugt mit 1 bis 6 Kohlenstoffatomen und insbesondere mit 1 bis 3 Kohlenstoffatomen eingesetzt.

Unter Alkenyl sind gemäß der vorliegenden Anmeldung verzweigte oder unverzweigte acyclische Kohlenwasserstoff-Reste zu verstehen, die mindestens eine Kohlenstoff-Kohlenstoff-Doppelbindung aufweisen. Geeignete Alkenyl-Reste sind beispielsweise 2-Propenyl, Vinyl, usw.. Bevorzugt weisen die Alkenyl-Reste 2 bis 50 Kohlenstoffatome, besonders bevorzugt 2 bis 20 Kohlenstoffatome, ganz besonders bevorzugt 2 bis 6 Kohlenstoffatome und insbesondere 2 bis 3 Kohlenstoffatome auf. Weiterhin sind unter dem Begriff Alkenyl solche Reste zu verstehen, die entweder eine cis- oder eine trans-Orientierung (alternativ E- oder Z-Orientierung) aufweisen.

Unter Alkinyl sind gemäß der vorliegenden Anmeldung verzweigte oder unverzweigte acyclische Kohlenwasserstoff-Reste zu verstehen, die mindestens eine Kohlenstoff-Kohlenstoff-Dreifachbindung aufweisen. Vorzugsweise weisen die Alkinyl-Reste 2 bis 50 Kohlenstoffatome, besonders bevorzugt 2 bis 20 Kohlenstoffatome, ganz besonders bevorzugt 1 bis 6 Kohlenstoffatome und insbesondere 2 bis 3 Kohlenstoffatome auf.

Unter substituiertem Alkyl, substituiertem Alkenyl und substituiertem Alkinyl sind Alkyl-Alkenyl- und Alkinyl-Reste zu verstehen, worin ein oder mehrere Wasserstoffatome, die an ein Kohlenstoffatom dieser Reste gebunden sind, durch eine andere Gruppe ersetzt sind. Beispiele für solche andere Gruppen sind Heteroatome, Halogen, Aryl, substituiertes Aryl, Cycloalkyl, Cycloalkenyl, substituiertes Cycloalkyl, substituiertes Cycloalkenyl und Kombinationen davon. Beispiele für geeignete substituierte Alkyl-Reste sind Benzyl, Trifluormethyl u. a.

Unter den Begriffen Heteroalkyl, Heteroalkenyl und Heteroalkinyl sind Alkyl- Alkenyl- und Alkinyl-Reste zu verstehen, worin ein oder mehrere der Kohlenstoffatome in der Kohlenstoffkette durch ein Heteroatom ausgewählt aus N, O und S ersetzt sind. Die Bindung zwischen dem Heteroatom und einem weiteren Kohlenstoffatom kann dabei gesättigt oder gegebenenfalls ungesättigt sein.

Unter Cycloalkyl sind gemäß der vorliegenden Anmeldung gesättigte cyclische nicht-aromatische Kohlenwasserstoff-Reste zu verstehen, die aus einem einzigen Ring oder mehreren kondensierten Ringen aufgebaut sind. Geeignete Cycloalkyl-Reste sind beispielsweise Cyclopentyl, Cyclohexyl, Cyclooctanyl, Bicyclooctyl usw.. Bevorzugt weisen die Cycloalkyl-Reste zwischen 3 und 50 Kohlenstoffatome, besonders bevorzugt zwischen 3 und 20 Kohlenstoffatome, ganz besonders bevorzugt zwischen 3 und 8 Kohlenstoffatome und insbesondere zwischen 3 und 6 Kohlenstoffatome auf.

Unter Cycloalkenyl sind gemäß der vorliegenden Anmeldung teilweise ungesättigte, cyclische nicht-aromatische Kohlenwasserstoff-Reste zu verstehen, die einen einzigen oder mehrere kondensierte Ringe aufweisen. Geeignete Cycloalkenyl-Reste sind beispielsweise Cyclopentenyl, Cyclohexenyl, Cyclooctenyl usw.. Bevorzugt weisen die Cycloalkenyl-Reste 3 bis 50 Kohlenstoffatome, besonders bevorzugt 3 bis 20 Kohlenstoffatome, ganz besonders bevorzugt 3 bis 8 Kohlenstoffatome und insbesondere 3 bis 6 Kohlenstoffatome auf.

Substituierte Cycloalkyl- und substituierte Cycloalkenyl-Reste sind Cycloalkyl- und Cycloalkenyl-Reste, worin ein oder mehrere Wasserstoffatome eines beliebigen Kohlenstoffatoms des Kohlenstoffrings durch eine andere Gruppe ersetzt sind. Solche andere Gruppen sind beispielsweise Halogen, Alkyl, Alkenyl, Alkinyl, substituiertes Alkyl, substituiertes Alkenyl, substituiertes Alkinyl, Aryl, substituiertes Aryl, Cycloalkyl, Cycloalkenyl, substituiertes Cycloalkyl, substituiertes Cycloalkenyl, ein aliphatischer heterocyclischer Rest, ein substituierter aliphatischer heterocyclischer Rest, Heteroaryl, substituiertes Heteroaryl, Alkoxy, Aryloxy, Boryl, Phosphino, Amino, Silyl, Thio, Seleno und Kombinationen davon. Beispiele für substituierte Cycloalkyl und Cycloalkenyl-Reste sind 4-Dimethylaminocyclohexyl, 4,5-Dibromocyclohept-4-enyl u.a..

Unter Aryl sind im Sinne der vorliegenden Anmeldung aromatische Reste zu verstehen, die einen einzelnen aromatischen Ring oder mehrere aromatische Ringe aufweisen, die kondensiert sind, über eine kovalente Bindung verknüpft sind oder durch eine geeignete Einheit, z.B. eine Methylen- oder Ethylen-Einheit verknüpft sind. Solche geeignete Einheiten können auch Carbonyl-Einheiten, wie in Benzophenol, oder Sauerstoff-Einheiten, wie in Diphenylether, oder Stickstoff-Einheiten, wie in Diphenylamin, sein. Der aromatische Ring, bzw. die aromatischen Ringe, sind beispielsweise Phenyl, Naphthyl, Diphenyl, Diphenylether, Diphenylamin und Benzophenon. Bevorzugt weisen die Aryl-Reste 6 bis 50 Kohlenstoffatome, besonders bevorzugt 6 bis 20 Kohlenstoffatome, ganz besonders bevorzugt 6 bis 8 Kohlenstoffatome auf.

Substituierte Aryl-Reste sind Aryl-Reste, worin ein oder mehrere Wasserstoffatome, die an Kohlenstoffatome des Aryl-Rests gebunden sind, durch eine oder mehrere andere Gruppen ersetzt sind. Geeignete andere Gruppen sind Alkyl, Alkenyl, Alkinyl, substituiertes Alkyl, substituiertes Alkenyl, substituiertes Alkinyl, Cycloalkyl, Cycloalkenyl, substituiertes Cycloalkyl, substituiertes Cycloalkenyl, Heterocyclo, substituiertes Hetereocyclo, Halogen, halogensubstituiertes Alkyl (z. B. $CF_3$), Hydroxy, Amino, Phosphino, Alkoxy, Thio und sowohl gesättigte als auch ungesättigte cyclische Kohlenwasserstoffe, die an den aromatischen Ring bzw. an die aromatischen Ringe kondensiert sein können oder durch eine Bindung verknüpft sein können, oder über eine geeignete Gruppe miteinander verknüpft sein können. Geeignete Gruppen sind bereits vorstehend erwähnt.

Unter Heterocyclo ist gemäß der vorliegenden Anmeldung ein gesättigter, teilweise ungesättigter oder ungesättigter cyclischer Rest zu verstehen, worin ein oder mehrere Kohlenstoffatome des Rests durch ein Heteroatom, z.B. N, O oder S ersetzt sind. Beispiele für Heterocyclo-Reste sind Piperazinyl, Morpholinyl, Tetrahydropyranyl, Tetrahydrofuranyl, Piperidinyl, Pyrolidinyl, Oxazolinyl, Pyridyl, Pyrazyl, Pyridazyl, Pyrimidyl.

Substituierte Heterocyclo-Reste sind solche Heterocyclo-Reste, worin ein oder mehrere Wasserstoffatome, die an eines der Ringatome gebunden sind, durch eine andere Gruppe ersetzt sind. Geeignete andere Gruppen sind Halogen, Alkyl, substituiertes Alkyl, Aryl, substituiertes Aryl, Heteroaryl, substituiertes Heteroaryl, Alkoxy, Aryloxy, Boryl, Phosphino, Amino, Silyl, Thio, Seleno und Kombinationen davon.

Unter Alkoxy-Resten sind Reste der allgemeinen Formel $-OZ^1$ zu verstehen, worin $Z^1$ ausgewählt ist aus Alkyl, substituiertem Alkyl, Cycloalkyl, substituiertem Cycloalkyl, Heterocycloalkyl, substituiertem Heterocycloalkyl, Silyl und Kombinationen davon. Geeignete Alkoxy-Reste sind beispielsweise Methoxy, Ethoxy, Benzyloxy, t-Butoxy usw.. Unter dem Begriff Aryloxy sind solche Reste der allgemeinen Formel $-OZ^1$ zu verstehen, worin $Z^1$ ausgewählt ist aus Aryl, substituiertem Aryl, Heteroaryl; substituiertem Heteroaryl und Kombinationen davon. Geeignete Aryloxy-Reste sind Phenoxy, substituiertes Phenoxy, 2-Pyridinoxy, 8-Chinolinoxy u.a..

Unter Amino-Resten sind Reste der allgemeinen Formel $-NZ^1Z^2$ zu verstehen, worin $Z^1$ und $Z^2$ unabhängig voneinander ausgewählt sind aus Wasserstoff, Alkyl, substituiertem Alkyl, Cycloalkyl, substituiertem Cycloalkyl, Heterocycloalkyl, substituiertem Heterocycloalkyl, Aryl, substituiertem Aryl, Heteroaryl, substituiertem Heteroaryl, Alkoxy, Aryloxy, Silyl und Kombinationen davon.

Bevorzugt in dem erfindungsgemäßen Aminierungsverfahren eingesetzte aromatische bzw. heteroaromatische Kohlenwasserstoffe sind ausgewählt aus Benzol, Diphenylmethan, Naphthalin, Anthracen, Toluol, Xylol, Phenol und Anilin sowie Pyridin, Pyrazin, Pyridazin, Pyrimidin und Chinolin. Dabei ist es auch möglich, Mischungen der genannten aromatischen bzw. heteroaromatischen Kohlenwasserstoffe einzusetzen. Besonders bevorzugt werden die aromatischen Kohlenwasserstoffe, Benzol, Naphthalin, Anthracen, Toluol, Xylol, Pyridin, Phenol und Anilin, ganz besonders bevorzugt Benzol, Toluol, und Pyridin eingesetzt.

Insbesondere bevorzugt wird Benzol in dem erfindungsgemäßen Aminierungsverfahren eingesetzt, so dass als Produkt Anilin entsteht.

Als Verbindung, durch die die Aminogruppe eingebracht wird, wird besonders bevorzugt Ammoniak eingesetzt. Dies bedeutet, dass erfindungsgemäß die Kohlenwasserstoffe, insbesondere das Benzol, besonders bevorzugt mit Ammoniak umgesetzt werden. Gegebenenfalls können auch Verbindungen Verwendung finden, die unter den Reaktionsbedingungen Ammoniak abspalten.

Für die Herstellung von Mono und Di-Alkyl-N,(N)-substituierten aromatischen Aminen, zum Beispiel von Mono- und oder Dimethylanilin, können auch Mono- und Di-Alkylamine, bevorzugt Mono- und Di(m)ethylamin, eingesetzt werden.

Die Reaktionsbedingungen in den erfindungsgemäßen Aminierungsverfahren sind unter anderem von dem zu aminierenden aromatischen Kohlenwasserstoff und dem eingesetzten Katalysator abhängig.

Die Aminierung, bevorzugt die Aminierung von Benzol, d.h. die Umsetzung von Benzol mit Ammoniak, erfolgt im Allgemeinen bei Temperaturen von 200 bis 800°C, bevorzugt 300 bis 700°C, besonders bevorzugt 325 bis 600°C und ganz besonders bevorzugt 350 bis 500°C.

Der Reaktionsdruck beträgt bei der Aminierung, bevorzugt bei der Aminierung von Benzol, d.h. die Umsetzung von Benzol mit Ammoniak, bevorzugt 1 bis 900 bar, besonders bevorzugt 1 bis 300 bar, insbesondere 5 bis 125 bar, insbesondere bevorzugt 15 bis 110 bar.

Die Verweilzeit beträgt in dem erfindungsgemäßen Aminierungsverfahren, bevorzugt bei der Aminierung von Benzol, im Allgemeinen 15 Minuten bis 8 Stunden, bevorzugt 15 Minuten bis 4 Stunden, besonders bevorzugt 15 Minuten bis 1 Stunde, bei der Durchführung in einem diskontinuierlichen Verfahren. Bei Durchführung in einem bevorzugten kontinuierlichen Verfahren beträgt die Verweilzeit im Allgemeinen 0,1 Sekunden bis 20 Minuten, bevorzugt 0,5 Sekunden bis 10 Minuten. Für die bevorzugten kontinuierlichen Verfahren bedeutet "Verweilzeit" in diesem Zusammenhang die Verweilzeit am Katalysator, für Festbettkatalysator somit die Verweilzeit im Katalysatorbett, für Wirbelschichtreaktoren wird der Syntheseteil des Reaktors (Teil des Reaktors, wo der Katalysator lokalisiert ist) betrachtet.

Die relative Menge des eingesetzten Kohlenwasserstoffs und der Aminkomponente ist abhängig von der durchgeführten Aminierungsreaktion und den Reaktionsbedingungen. Im Allgemeinen werden mindestens stöchiometrische Mengen des Kohlenwasserstoffs und der Aminkomponente eingesetzt. Üblicherweise ist es jedoch bevorzugt, einen der Reaktionspartner im stöchiometrischen Überschuss einzusetzen, um eine Gleichgewichtsverschiebung auf die Seite des gewünschten Produkts und somit einen höheren Umsatz zu erreichen. Bevorzugt wird die Aminkomponente im stöchiometrischen Überschuss eingesetzt.

Das erfindungsgemäße Aminierungsverfahren kann kontinuierlich, diskontinuierlich oder semi-kontinuierlich durchgeführt werden. Geeignete Reaktoren sind somit sowohl Rührkessel-Reaktoren als auch Rohr-Reaktoren. Typische Reaktoren sind beispielsweise Hochdruck-Rührkessel-Reaktoren, Autoklaven, Festbettreaktoren, Wirbelschichtreaktoren, Wanderbetten, zirkulierende Wirbelschichten, Salzbadreaktoren, Plattenwärmetauscher als Reaktoren, Hordenreaktoren mit mehreren Horden mit/oder ohne Wärmetausch bzw. Abzug/Zufuhr von Teilströmen zwischen den Horden, in möglichen Ausführungen als Radialstrom- oder Axialstromreaktoren, kontinuierlich gerührte Kessel, Blasenreaktoren usw., wobei jeweils der für die gewünschten Reaktionsbedingungen (wie Temperatur, Druck und Verweilzeit) geeignete Reaktor eingesetzt wird. Die Reaktoren können jeweils als einzelner Reaktor (single reactor), als Serie von einzelnen Reaktoren und/oder in Form von zwei oder mehr parallelen Reaktoren eingesetzt werden. Die Reaktoren können in einer AB Fahrweise betrieben werden (alterierende Fahrweise). Das erfindungsgemäße Verfahren kann als Batch-Reaktion, semikontinuierliche Reaktion oder kontinuierliche Reaktion durchgeführt werden. Der spezielle Reaktoraufbau und die Durchführung der Reaktion können in Abhängigkeit von dem durchzuführenden Aminierungsverfahren, dem Aggregatzustand des zu aminierenden aromatischen Kohlenwasserstoffs, den erforderlichen Reaktionszeiten und der Natur des eingesetzten stickstoffhaltigen Katalysators variieren. Bevorzugt wird das erfindungsgemäße Verfahren zur Direktaminierung in einem Hochdruck-Rührkessel-Reaktor, Festbettreaktor oder Wirbelschichtreaktor durchgeführt.

In einer besonders bevorzugten Ausführungsform wird bei der Aminierung von Benzol zu Anilin Festbett- oder Wirbelschicht-Reaktor eingesetzt, wobei eine interne Anordnung der Membrane erfolgt und somit im Syntheseteil Wasserstoff entfernt wird. Ein weiterer Vorteil der Membran, welche mittels eines Spülstroms durchströmt werden kann, ist eine gute Wärmekontrolle des Reaktors: Reaktionswärme kann zugegeben oder bevorzugt abgeführt werden durch heizen oder abkühlen des Spülstroms.

Der Kohlenwasserstoff und die Aminkomponente können in gasförmiger oder flüssiger Form in die Reaktionszone des jeweiligen Reaktors gegeben werden. Die bevorzugte Phase ist jeweils abhängig von der durchgeführten Aminierung sowie dem eingesetzten Reaktor. In einer bevorzugten Ausführungsform, zum Beispiel bei der Herstellung von Anilin aus Benzol, liegen Benzol und Ammoniak bevorzugt als gasförmige Reaktanden in der Reaktionszone vor. Typischerweise wird Benzol dabei als Flüssigkeit zugeführt, die aufgeheizt wird und verdampft, wobei ein Gas gebildet wird, während Ammoniak entweder in gasförmiger Form oder in überkritischer Phase in der Reaktionszone vorliegt. Es ist ebenfalls möglich, dass Benzol zumindest zusammen mit Ammoniak in überkritischer Phase vorliegt.

Der Kohlenwasserstoff und die Aminkomponente können gemeinsam in die Reaktionszone des Reaktors gegeben werden, zum Beispiel als vorgemischter Reaktandenstrom, oder getrennt. Bei einer getrennten Zugabe können der Kohlenwasserstoff und die Aminkomponente entweder gleichzeitig, zeitversetzt oder nacheinander in die Reaktionszone des Reaktors gegeben werden. Bevorzugt erfolgen die Zugabe der Aminkomponente und die Zugabe des Kohlenwasserstoffs zeitversetzt.

Bevorzugt kann man zusätzlich zu der chemischen Umwandlung des Wasserstoffes diesen auch physikalisch aus dem Reaktionsgemisch entfernen.

Unter dem Ausdruck "physikalisch entfernen" ist zu verstehen, dass der Wasserstoff physisch und bevorzugt selektiv aus dem Reaktionsgemisch entweicht.

Bevorzugt erfolgt die physikalische Entfernung des Wasserstoffs aus dem Reaktionsgemisch, indem man Wasserstoff durch eine Wasserstoff- permeable, bevorzugt Wasserstoff-selektive Membran aus dem Reaktionsgemisch entfernt, bevorzugt indem der Wasserstoff aus dem Reaktionsgemisch durch die Membran diffundiert. Die Diffusion des Wasserstoffs ist dabei bevorzugt getrieben durch das Konzentrationsgefälle zwischen dem Reaktionssystem (Retentatseite), in dem bevorzugt durch die Reaktion von Benzol mit Ammoniak Wasserstoff entsteht, und dem auf der anderen Seite der Membran befindlichen Raum (Permeatseite). Der auf die Permeatseite diffundierte Wasserstoff kann dort bevorzugt durch Abtransport, beispielsweise mittels Gasstrom oder Unterdruck, und/oder durch chemische Reaktion, beispielsweise durch Reduktion einer organische Verbindung in der Gasphase, zum Beispiel Benzol zu Cyclohexan oder unter Bildung von Wasser, bevorzugt mit einem sauerstoffhaltigen Gas, wie zum Beispiel Luft, bevorzugt durch

katalysierte Reaktion mit Sauerstoff und/oder Luft, abgereichert, d.h. entfernt werden. Dadurch wird das Konzentrationsgefälle zwischen Retentatseite und Permeatseite, das die Diffusion treibt, aufrechterhalten oder erhöht.

Bevorzugt kann die Wasserstoff-permeable Membran Teil eines Reaktor sein und beispielsweise den Reaktionsraum, in dem bevorzugt Benzol mit Ammoniak umgesetzt wird, zumindest teilweise begrenzen. Bevorzugt kann somit das erfindungsgemäße Verfahren derart erfolgen, dass die Aminierung, bevorzugt die Umsetzung von Benzol mit Ammoniak, in einem Membranreaktor mit integrierter Wasserstoffabtrennung durch eine Wasserstoff- permeable Membran erfolgt.

Bevorzugt weist die Membran eine Permeanz für Wasserstoff von größer 10 $m^3$ /($m^2$ x h x bar"), besonders bevorzugt > 50 $m^3$ /($m^2$ x h x bar") auf, wobei theoretisch n=0,5 und real zwischen 0,5 und 0,6 bedeutet, somit bevorzugt n zwischen 0,5 und 0,6, besonders bevorzugt n = 0,5. Die Permeanz (P) kann aus dem Wasserstofffluss (in $m^3$/($m^2$,h) und den Wasserstoffpartialdrücken berechnet werden:

$$P = \frac{Wasserstofffluss}{P^n_{Retentat} - P^n_{Permeat}}$$

Die Membran ist bevorzugt möglichst selektiv für Wasserstoff. D.h., dass die Membran bevorzugt dicht ist und besonders bevorzugt eine $H_2$ /$N_2$ Selektivität > 1000 aufweist. Durch Einsatz solcher Membranen wird gewährleistet, dass nur ein möglichst geringer Anteil an Edukt (Kohlenwasserstoff, Ammoniak) und/oder Produkt (insbesondere Anilin) auf die Permeatseite der Membran gelangt. Für die bevorzugten Pd und Pd-Legierte Membrane können die Edukte und Kohlenwasserstoffe nicht durch die Membran diffundieren.

Die Membran weist bevorzugt eine Dicke zwischen 0,1 $\mu$m und 25 $\mu$m, besonders bevorzugt zwischen 0,5 $\mu$m und 10 $\mu$m, ganz besonders bevorzugt zwischen 1 $\mu$m und 5 $\mu$m auf.

Die Membran kann selbsttragend aufgebaut sein, Aufgrund der im Regelfall sehr hohen Materialkosten kann es von Vorteil sein, die eigentliche Membran auf einer porösen keramischen und/oder metallischen Stützschicht zu fixieren ("Composit"- Membran). Dies bietet zudem den Vorteil, dass die Membran stabilisiert wird und außerdem geringe Schichtdicken ermöglicht werden. Typische Membranen können zum Beispiel bei NGK in Japan oder bei Johnson Matthey gekauft werden.

Als geeignete Membranen kommen beispielsweise mesoporöse anorganische Membranen, mikroporöse anorganische Membranen, Polymer-Membranen, Membranen auf Basis von Metallen der 4. oder 5. Nebengruppe beschichtet mit Palladium, nanokristalline Metallmembranen, gemischtleitende Membranen und bevorzugt Membranen auf der Basis von Palladium oder Palladiumlegierungen in Frage.

Mesoporöse anorganische Membranen sind beispielsweise solche mit einer Porengröße kleiner 50 nm, z.B. solche auf Basis von $Al_2O_3$.

Mikroporöse anorganische Membranen sind beispielsweise solche mit einer Porengröße kleiner 2 nm, z.B. solche auf Basis von keramischen- oder Kohlenstoff-Molekularsieben. Als keramische Molekularsieb-Membranen kommen zeolithische, beispielsweise solche vom MFI-Typ (ZSM-5 oder Silicalit), die gegebenenfalls auf $Al_2O_3$ geträgert sein können, und amorphe, z.B. solche aus $SiO_2$ in Betracht. Kohlenstoff-Molekularsieb-Membranen lassen sich durch Carbonisierung von organischen Polymeren, z.B. Polyimid herstellen.

Polymer-Membranen sind "Composit"-Membranen aus einer dichten wasserstoffselektiven Polymerschicht auf einem anorganischen Träger.

Als Membranen auf Basis von Metallen der 4. und/oder 5. Nebengruppe, die mit Palladium beschichtet sind, kommen beispielsweise solche in Betracht, bei denen auf einem nichtporösen Träger auf Basis von Nichtedelmetall, bevorzugt Vanadium, Niob und/oder Tantal, ein oder bevorzugt zwei Schichten aus Palladium oder Palladiumlegierungen vorliegen.

In Frage kommen auch Membranen aus TiN, nanokristalline Metallschichten, beispielsweise auf $Al_2O_3$ geträgertes Platin oder Ruthenium oder amorphe Membranen.

Geeignet sind ferner gemischt leitende Membranen, die sowohl elektronische als auch ionische Leitfähigkeit aufweisen.

Bevorzugt sind allerdings Membranen, die auf Palladium oder Palladiumlegierungen basieren. Als Legierungen kommen insbesondere Legierungen von Palladium mit Silber und/oder Kupfer in Betracht. Dabei sind besonders bevorzugt Membran auf Basis einer Legierung enthaltend Palladium und zwischen 23 Gew.-% und 25 Gew.-% Silber, bezogen auf das Gesamtgewicht der Legierung. Die Legierung enthält dabei besonders bevorzugt zwischen 75 Gew.-% und 77 Gew.-% Palladium, bezogen auf das Gesamtgewicht der Legierung. Besonders bevorzugt sind ferner Membranen auf Basis einer Legierung enthaltend Palladium und zwischen 34 Gew.-% und 46 Gew.-% Kupfer, bezogen auf das Gesamtgewicht der Legierung. Die Legierung enthält dabei besonders bevorzugt zwischen 54 Gew.-% und 66

Gew.-% Palladium, bezogen auf das Gesamtgewicht der Legierung. Die Palladium- bzw. die Palladiumlegierung- Membranen können weiter mit Seltenerdmetalle, wie z.B. Gadolinium dotiert werden. Dabei können die Palladium- bzw. die Palladiumlegierung- Membranen übliche weitere Metalle enthalten in üblichen Mengen, die die Permeabilität und Selektivität für Wasserstoff nicht oder zumindest nicht wesentlich beeinträchtigen. Auch diese bevorzugten Membranen können auf porösen Unterstrukturen vorliegen, die die eigentliche Membran fixieren und stabilisieren. Die poröse Unterstruktur kann beispielsweise auf Keramik, Metall oder einem organischen Polymeren basieren, z.B. $TiO_2$ und/oder $Al_2O_3$. Die Herstellung der bevorzugt dichten Metallmembran (bevorzugt Palladium oder Palladiumlegierung) auf einem porösen Träger ist allgemein bekannt und kann beispielsweise durch galvanisches Aufbringen, Sputtern, CVD (Chemical Vapor Deposition) oder bevorzugt allgemein bekannte stromlose nasschemische Beschichtung erfolgen.

Wie bereits dargestellt trennt die Membran bevorzugt die Retentatseite (Reaktionsseite) von der Permeatseite, wobei der auf der Retentatseite gebildete Wasserstoff durch die Membran auf die Permeatseite gelangt, wo der Wasserstoff durch Reaktion, bevorzugt Reaktion mit Sauerstoff oder einem sauerstoffhaltigen Strom, wie zum Beispiel Luft, bevorzugt in Gegenwart von Katalysatoren, und/oder Stofftransport, bevorzugt mittels eines Gasstroms ("Sweepgas" oder Spühlgas) entfernt wird. Die selektive Entfernung des Wasserstoffs an der Permeatseite ermöglicht eine deutliche weitere Absenkung des Wasserstoffpartialdruckes an der Retentatseite der Membran (= Reaktionsseite) und ermöglicht so, die gewünschten hohen Benzolumsätze (>5 mol %, >10 mol %, >20 mol % bezogen auf die zugegebene Menge Benzol) bei höher Anilinselektivität (>95 %, >98 %, >99 %, Anilinselektivität: mol Anilin/ Summe aller gebildeten Produkte in mol (=Benzolumsatz)).

Im Anschluss an die Aminierung kann die Isolierung des gewünschten Produktes nach dem Fachmann bekannten Verfahren erfolgen.

Beispiel 1: Herstellung von Aminierungkatalysator (i)

Die Herstellung des Katalysators erfolgt gemäß DE-A 44 28 004:

Eine wässrige Lösung aus Nickelnitrat, Kupfernitrat und Zirconacetat, die 4,48 Gew.-% Ni (berechnet als NiO), 1,52 Gew.-% Cu (berechnet als CuO und 2,28 Gew.-% Zr (berechnet als $ZrO_2$) enthält, wird gleichzeitig in einem Rührgefäß in einem konstanten Strom mit einer 20 %igen wässrigen Natriumcarbonatlösung bei einer Temperatur von 70°C so gefällt, dass der mit einer Glaselektrode gemessene pH-Wert von 7,0 aufrechterhalten wird. Die erhaltene Suspension wird filtriert und der Filterkuchen mit voll entsalztem Wasser gewaschen bis die elektrische Leitfähigkeit des Filtrats ca. 20 $\mu$S beträgt. Dann wird in den noch feuchten Filterkuchen so viel Ammoniümheptamolybdat eingearbeitet, dass das nachfolgend angegebene Oxidgemisch erhalten wird. Danach wird der Filterkuchen bei einer Temperatur von 150°C in einem Trockenschrank oder einem Sprühtrockner getrocknet. Das auf diese Weise erhaltene Hydroxid-Carbonat-Gemisch wird nun bei einer Temperatur von 430 bis 460°C über einen Zeitraum von 4 Stunden getempert. Die so hergestellte oxidische Spezies hat die Zusammensetzung: 50 Gew.-% NiO, 17 Gew.-% CuO, 1,5 Gew.-% $MoO_3$ und 31,5 Gew.-% $ZrO_2$. Die Reduktion wird bei 290°C durchgeführt, wobei die Aufheizungsrate 3°C/Minute beträgt. Zuerst wurde 50 Minuten mit 10% $H_2$ in $N_2$ reduziert, anschließend 20 Minuten mit 25 % $H_2$ in $N_2$, dann 10 Minuten mit 50 % $H_2$ in $N_2$, dann 10 Minuten mit 75 % $H_2$ in $N_2$ und schließlich 3 Stunden mit 100% $H_2$. Bei den %-Angaben handelt es sich jeweils um Volumen-%. Die Passivierung der reduzierten oxidischen Spezies wird bei Raumtemperatur in verdünnter Luft (Luft in $N_2$ mit einem $O_2$-Gehalt von maximal 5 Vol.-%) durchgeführt.

Beispiel 2: Herstellung von Aminierungkatalysator (i)

Es wird eine Lösung aus 132 mL Wasser, 27,84g $Ni(NO_3)_2 * 6H_2O$, 61,68g $Cu(NO_3)_2 * 2\frac{1}{2}H_2O$ und 3,52g $AgNO_3$ hergestellt. 200g Trägermaterial $ZrO_2$ wird mit der Hälfte der Tränklösung getränkt und anschließend bei 120°C 12h an der Luft getrocknet. Danach wird der Katalysator mit dem Rest der Tränklösung getränkt und bei 120°C 12 h an der Luft getrocknet. Anschließend wird der Katalysator 4h bei 400°C kalziniert. Der so entstehende Katalysator enthält 7,3 Gew.-% Cu, 2,4 Gew.-% Ni und 0,98 Gew.-% Ag.

Beispiel 3: Herstellung von Oxidationskatalysator (ii)

Pt getränkt auf $Al_2O_3$

Pt-Nitrat (die Menge an Pt-Nitrat ergeben sich aus der Zusammensetzung des erhaltenen Katalysatorsystems) wurde in destilliertem Wasser gelöst, so dass eine 2 Gew.-%-ige wässrige Lösung entsteht. Ein $Al_2O_3$-Trägermaterial wird mit dieser Lösung getränkt. Nach Trocknung bei 120°C und Kalzinierung für 4 Stunden bei 450°C erhält man ein Katalysatorsystem mit 0,001 - 0,1 Gew.-% Pt, wobei Pt gemeinsam mit dem Trägermaterial 100 Gew.-% ergibt.

## Patentansprüche

1. Verfahren zur Aminierung von Kohlenwasserstoffen mit Ammoniak in Gegenwart von Katalysator (i), der die Aminierung katalysiert, wobei die Kohlenwasserstoffe beliebige Kohlenwasserstoffe sein können, wie aromatischen Kohelnwasserstoffe, aliphatische Kohlenwasserstoffe und cycloaliphatische Kohlenwasserstoffe, die beliebig substituiert sein können und innerhalb ihrer Kette bzw. ihres Rings oder ihrer Ringe Heteroatome und Doppel- oder Dreifachbindungen aufweisen können, -**dadurch gekennzeichnet, dass** man in einem ersten Reaktionsraum diesen Kohlenwasserstoffe mit Ammoniak in Gegenwart des Katalysators (i) umsetzt, danach das Oxidationsmittel dem Reaktionsgemisch zuführt und in einem anschließenden Reaktionsraum das Oxidationsmittel mit dem bei der Aminierung entstehenden Wasserstoff in Gegenwart des Katalysators (ii), der diese Umsetzung mit Wasserstoff katalysiert, umsetzt, wobei man während oder nach der Wasserstoffoxidation Kohlenwasserstoff mit Ammoniak in Gegenwart des Katalysators (i) umsetzt.

2. Verfahren gemäß Anspruch 1, **dadurch gekennzeichnet, dass** die Katalysatoren (i) und (ii) jeweils in getrennten Katalysatorbetten vorliegen, das Oxidationsmittel dem Reaktionsgemisch vor einem Katalysatorbett mit dem Katalysator (ii) zugeführt wird und auf das Katalysatorbett mit dem Katalysator (ii) ein Katalysatorbett mit dem Katalysator (i) folgt.

3. Verfahren gemäß Anspruch 1, **dadurch gekennzeichnet, dass** man als Katalysator (i) Verbindungen einsetzt, die Ni, Co, Fe, Cu oder Kombinationen dieser Elemente enthalten.

4. Verfahren gemäß Anspruch 1, **dadurch gekennzeichnet, dass** man als Katalysator (i) Verbindungen einsetzt, die Ni-Cu-X, Fe-Cu-X und/oder Co-Cu-X enthalten, wobei X Ag oder Mo darstellt.

5. Verfahren gemäß Anspruch 1, **dadurch gekennzeichnet, dass** man als Katalysator (ii) Pt und/oder Pd und Ag enthaltende Verbindungen einsetzt.

6. Verfahren gemäß Anspruch 1 oder 4, **dadurch gekennzeichnet, dass** in dem Katalysator (i) der Gewichtsanteil der Elemente Ni, Co und Fe zusammen zwischen 0,1 Gew.-% und 75 Gew.-% und der Gewichtsanteil an Cu zwischen 0,1 Gew.-% und 75 Gew.-%, jeweils bezogen auf das Gesamtgewicht des Katalysators (i), beträgt.

7. Verfahren gemäß Anspruch 4, **dadurch gekennzeichnet, dass** der Gewichtsanteil des Dotierelements X an dem Gesamtgewicht des Katalysators (i) zwischen 0,01 Gew.-% und 8 Gew.-%, bezogen auf das Gesamtgewicht des Katalysators (i), beträgt.

8. Verfahren gemäß Anspruch 5, **dadurch gekennzeichnet, dass** in dem Katalysator (ii) der Gewichtsanteil an Pt zwischen 0,0001 Gew.-% und 1 Gew.-%, bezogen auf das Gesamtgewicht des Katalysators (ii), beträgt.

9. Verfahren gemäß Anspruch 1, **dadurch gekennzeichnet, dass** das Katalysatorbett enthaltend Katalysatoren (i) und (ii) mit einer Belastung von 0,1 bis 5 kg Kohlenwasserstoff pro Liter Katalysatorbett und pro Stunde belastet wird.

10. Verfahren gemäß Anspruch 1, **dadurch gekennzeichnet, dass** man die Aminierung kontinuierlich durchführt.

11. Verfahren gemäß Anspruch 1, **dadurch gekennzeichnet, dass** man die Aminierung bei Temperaturen zwischen 200 und 800°C durchführt.

12. Verfahren gemäß Anspruch 1, **dadurch gekennzeichnet, dass** man die Aminierung bei Drücken zwischen 1 bis 900 bar durchführt.

13. Verfahren gemäß Anspruch 1, **dadurch gekennzeichnet, dass** man Wasserstoff aus dem Reaktionsgemisch *zusätzlich* physikalisch entfernt.

14. Verfahren gemäß Anspruch 13, **dadurch gekennzeichnet, dass** die Aminierung in einem Membranreaktor mit integrierter Wasserstoffabtrennung durch eine Wasserstoff- permeable Membran erfolgt.

## Claims

1. A process for aminating hydrocarbons with ammonia in the presence of catalyst (i) which catalyzes the amination, where the hydrocarbons may be any hydrocarbons, such as aromatic hydrocarbons, aliphatic hydrocarbons and cycloaliphatic hydrocarbons, which may have any substitution and may have heteroatoms and double or triple bonds within their chain or their ring or their rings, which comprises reacting said hydrocarbons with ammonia in the presence of the catalyst (i) in a first reaction chamber, then supplying the oxidizing agent to the reaction mixture and, in a subsequent reaction chamber, reacting the oxidizing agent with the hydrogen formed in the amination in the presence of the catalyst (ii) which catalyzes this reaction with hydrogen, hydrocarbon reacting with ammonia in the presence of the catalyst (i) during or after the hydrogen oxidation.

2. The process according to claim 1, wherein the catalysts (i) and (ii) are each present in separate catalyst beds, the oxidizing agent is supplied to the reaction mixture upstream of a catalyst bed comprising the catalyst (ii), and the catalyst bed comprising the catalyst (ii) is followed by a catalyst bed comprising the catalyst (i).

3. The process according to claim 1, wherein the catalyst (i) used comprises compounds which comprise Ni, Co, Fe, Cu or combinations of these elements.

4. The process according to claim 1, wherein the catalyst (i) used comprises compounds which comprise Ni-Cu-X, Fe-Cu-X and/or Co-Cu-X, where X is Ag or Mo.

5. The process according to claim 1, wherein the catalyst (ii) used comprises compounds comprising Pt and/or Pd and Ag.

6. The process according to claim 1 or 4, wherein the proportion by weight of the elements Ni, Co and Fe together in catalyst (i) is between 0.1% by weight and 75% by weight, and the proportion by weight of Cu is between 0.1% by weight and 75% by weight, based in each case on the total weight of catalyst (i) .

7. The process according to claim 4, wherein the proportion by weight of the doping element X in the total weight of the catalyst (i) is between 0.01% by weight and 8% by weight based on the total weight of the catalyst (i).

8. The process according to claim 5 wherein the proportion by weight of Pt in catalyst (ii) is between 0.0001% by weight and 1% by weight, based on the total weight of catalyst (ii).

9. The process according to claim 1, wherein the catalyst bed comprising catalysts (i) and (ii) is loaded with an hourly space velocity of from 0.1 to 5 kg of hydrocarbon per liter of catalyst bed and per hour.

10. The process according to claim 1, wherein the amination is performed continuously.

11. The process according to claim 1, wherein the amination is performed at temperatures between 200 and 800°C.

12. The process according to claim 1, wherein the amination is performed at pressures between 1 to 900 bar.

13. The process according to claim 1, wherein hydrogen is additionally removed physically from the reaction mixture.

14. The process according to claim 13, wherein the amination is effected in a membrane reactor with integrated hydrogen removal by a hydrogen-permeable membrane.

## Revendications

1. Procédé d'amination d'hydrocarbures avec de l'ammoniac en présence d'un catalyseur (i), qui catalyse l'amination, les hydrocarbures pouvant être des hydrocarbures quelconques, tels que des hydrocarbures aromatiques, des hydrocarbures aliphatiques et des hydrocarbures cycloaliphatiques, qui peuvent être substitués de manière quelconque et présenter dans leur chaîne ou, selon le cas, leur cycle ou leurs cycles des hétéroatomes et des doubles ou triples liaisons, **caractérisé en ce qu'**on transforme dans un premier espace de réaction ces hydrocarbures avec de l'ammoniac en présence du catalyseur(i), puis on introduit l'oxydant dans le mélange réactionnel et on transforme, dans un espace de réaction consécutif, l'oxydant avec l'hydrogène formé lors de l'amination en présence

du catalyseur (ii), qui catalyse cette transformation avec l'hydrogène, en transformant pendant ou après l'oxydation avec l'hydrogène l'hydrocarbure avec de l'ammoniac en présence du catalyseur (i).

**2.** Procédé selon la revendication 1, **caractérisé en ce que** les catalyseurs (i) et (ii) se trouvent à chaque fois dans des lits de catalyseur séparés, l'oxydant est introduit dans le mélange réactionnel en amont d'un lit de catalyseur contenant le catalyseur (ii) et le lit de catalyseur contenant le catalyseur (ii) est suivi d'un lit de catalyseur contenant le catalyseur (i).

**3.** Procédé selon la revendication 1, **caractérisé en ce qu'**on utilise comme catalyseur (i) des composés qui contiennent Ni, Co, Fe, Cu ou des combinaisons de ces éléments.

**4.** Procédé selon la revendication 1, **caractérisé en ce qu'**on utilise comme catalyseur (i) des composés qui contiennent Ni-Cu-X, Fe-Cu-X et/ou Co-Cu-X, X représentant Ag ou Mo.

**5.** Procédé selon la revendication 1, **caractérisé en ce qu'**on utilise comme catalyseur (ii) des composés contenant Pt et/ou Pd et Ag.

**6.** Procédé selon la revendication 1 ou 4, **caractérisé en ce que**, dans le catalyseur (i), la proportion en poids des éléments Ni, Co et Fe ensemble se situe entre 0,1% en poids et 75% en poids et la proportion en poids de Cu se situe entre 0,1% en poids et 75% en poids, à chaque fois par rapport au poids total du catalyseur (i).

**7.** Procédé selon la revendication 4, **caractérisé en ce que** la proportion en poids de l'élément de dopage X par rapport au poids total du catalyseur (i) se situe entre 0,01% en poids et 8% en poids, par rapport au poids total du catalyseur (i).

**8.** Procédé selon la revendication 5, **caractérisé en ce que**, dans le catalyseur (ii), la proportion en poids de Pt se situe entre 0,0001% en poids et 1% en poids, par rapport au poids total du catalyseur (ii).

**9.** Procédé selon la revendication 1, **caractérisé en ce que** le lit de catalyseur contenant les catalyseurs (i) et (ii) est chargé avec 0,1 à 5 kg d'hydrocarbure par litre de lit de catalyseur et par heure.

**10.** Procédé selon la revendication 1, **caractérisé en ce qu'**on réalise l'amination en continu.

**11.** Procédé selon la revendication 1, **caractérisé en ce qu'**on réalise l'amination à des températures entre 200 à 800°C.

**12.** Procédé selon la revendication 1, **caractérisé en ce qu'**on réalise l'amination à des pressions entre 1 à 900 bars.

**13.** Procédé selon la revendication 1, **caractérisé en ce qu'**on élimine en outre physiquement de l'hydrogène du mélange réactionnel.

**14.** Procédé selon la revendication 13, **caractérisé en ce que** l'amination a lieu dans un réacteur à membrane avec une séparation intégrée d'hydrogène par une membrane perméable à l'hydrogène.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- CN 1555921 A
- CA 553988
- US 3919155 A
- US 3929889 A
- US 4001260 A
- US 4031106 A
- DE 19634110
- WO 0009473 A
- WO 9910311 A
- WO 0132600 A
- WO 0069804 A
- DE 4428004 A

### In der Beschreibung aufgeführte Nicht-Patentliteratur

- *Berichte,* vol. 50, 541-546